# DEMANDE DE BREVET EUROPEEN

(11) **EP 1 604 641 A2**
(43) Date de publication de la demande: **14.12.2005**
(21) Numéro de dépôt: 05291090.8
(22) Date de dépôt: 20.05.2005
(51) Int. Cl.: A61K 7/075

(54) **Procédé de lavage des cheveux frisés ou crépus**

(30) Priorité: 11.06.2004 FR 0451160
(71) Demandeur: L'OREAL, 75008 Paris (FR)
(72) Inventeur: Saint-Leger Didier, 92110 Clichy (FR)
(74) Mandataire: Le Blainvaux Bellegarde, Françoise

(57) **Abrégé**

La présente invention a pour objet l'utilisation d'une composition détergente liquide pour nettoyer les cheveux frisés ou crépus et le cuir chevelu, et un procédé nettoyage de ces derniers utilisant une composition détergente liquide.

## Description

La présente invention a pour objet l'utilisation d'une composition capillaire liquide destinée au nettoyage des cheveux frisés et/ou crépus, et un procédé de nettoyage de ces derniers utilisant cette composition.

Les chevelures ont tendance à perdre certaines de leurs qualités sous l'action de facteurs tels que le regraissage naturels, la sueur, l'élimination de squames, la pollution, l'humidité et autres. Ces facteurs nuisent à l'aspect visuel et au toucher des cheveux. Ainsi, le regraissage (et éventuellement la pollution) alourdit les cheveux, lesquels ont tendance à se mettre en paquets. Les cheveux sont alors difficiles à coiffer, ils ont une brillance grasse désagréable et ont un toucher ciré également désagréable.
L'ampleur des conséquences de ces facteurs, presque tous inévitables, est très variable. Elle dépend par exemple de la qualité des cheveux, de leurs longueurs et de la coiffure adoptée.

Quoiqu'il en soit, pour lutter contre ces désagréments, on utilise des shampooings, compositions à base de tensio-actifs détergents de différentes natures, dont l'effet mouillant permet la mise en émulsion des composés gras (le sébum pour l'essentiel) puis leur élimination au cours du rinçage. Ces shampooings permettent généralement d'éliminer aussi les salissures et les pellicules.

Dans la grande majorité des cas, de telles formules, bien qu'efficaces et plaisantes, sont de nature physico chimique épaisse ou visqueuse.
Cette grande consistance ou forte viscosité (variant généralement de 2000 à 20000 Centipoises, déterminée par un Rhéomètre rotatif à la vitesse de 1s⁻¹, 1 Centipoise étant égal à 1 Milli Pascal/s) fait que les produits commercialisés sont tous sous forme d'un packaging type flacon souple, le produit s'écoulant plus ou moins lentement par gravité, selon sa viscosité propre, ou en le forçant par pression des doigts sur les parois souples du packaging.
Sur des chevelures de formes raides, courbes ou ondulées, telles qu'on peut les trouver dans les groupes humains Asiatiques ou Caucasiens, cette forte consistance de formule n'empêche nullement un démarrage de la mousse assez rapide et son étalement aisé sur le cuir chevelu et les cheveux. En revanche, sur des chevelures fortement bouclées ou crépues, telles que celles des populations d'ethnies Africaines ou leurs ethnies affiliées comme, par exemple, les ethnies Afro-Américaines ou Antillaises, cette consistance entraîne non seulement un démarrage de mousse particulièrement tardif et lent, mais aussi un nettoyage du cuir chevelu peu efficace par manque de contact intime entre la base lavante épaisse et le cuir chevelu.
On trouvera une revue de ces problèmes spécifiques de ces ethnies humaines dans le livre « Dermatologie sur Peau noire en France métropolitaine » des Drs Fitoussi et Sulimovic, Médecine-Sciences-Flammarion, 2003 ou dans l'article « Ethnic hair updates : Past and Present », A J Mc Michael, J Am Acad Dermatol ; 48,127-133,2003.

La présente invention a notamment pour but de résoudre les problèmes ci-dessus.

Plus précisément encore, la présente invention vise à proposer un procédé de nettoyage des cheveux rapide qui conduise, en fin d'opération, à des cheveux présentant un toucher et un aspect visuel propre.

Or, à la suite d'importantes recherches menées sur la question, il a maintenant été trouvé par la Demanderesse, et ceci de façon tout à fait inattendue et surprenante, que ce but, et d'autres, pouvaient être atteints en mettant en oeuvre des compositions cosmétiques liquides contenant dans un support cosmétiquement acceptable, comprenant au moins 5% d'au moins un tensioactif détergent. Cette découverte est à la base de la présente invention.

Le lavage des cheveux et du cuir chevelu est non seulement rapide mais s'avère complet, i.e débarrassé de sébum, d'une majeure partie de la flore microbienne de surface, des cellules mortes ainsi que des résidus de traitements capillaires précédents.

La présente invention a pour objet un procédé de nettoyage des cheveux bouclés, frisés ou crépus du cuir chevelu correspondant, ledit procédé étant caractérisé par le fait qu'il comprend les étapes suivantes :
(i) on applique sur les cheveux secs ou humides une composition liquide comprenant dans un milieu cosmétiquement acceptable au moins 5% d'au moins un tensioactif détergent,
(ii) on masse les cheveux et le cuir chevelu éventuellement à l'aide des doigts ,
(iii) on rince les cheveux avec de l'eau,
la viscosité de la composition allant de 1 à 100 mPa/s.

La présente invention a pour objet l'utilisation d'une composition détergente liquide pour nettoyer les cheveux frisés ou crépus et le cuir chevelu.

L'invention consiste aussi en une utilisation des compositions de l'invention sur des cheveux bouclés, frisés ou crépus pour obtenir un bon démarrage de mousse et/ou une meilleure réduction des quantités résiduelles de sébum et/ou une meilleure réduction de la flore résidente.

La viscosité des compositions utilisées par le dit procédé se situe avantageusement entre 1 et 100 Centipoises (mPa/s), préférentiellement entre 2 et 20 Centipoises (mPa/s), et plus préférentiellement encore entre 5 et 15 Centipoises (mPa/s).

Les viscosité sont mesurée à 25°C à l'aide d'un Rhéomètre rotatif à la vitesse de 1s⁻¹.

Le ou les tensioactifs détergents sont choisis, seuls ou en mélanges, au sein des tensioactifs anioniques, amphotères, non ioniques et zwittérioniques.

Ainsi, selon l'invention, les tensioactifs détergents représentent de 5 % à 50 % en poids, de préférence de 6 % à 30 % en poids, et encore plus préférentiellement de 8 % à 25 % en poids, du poids total de la composition finale.

Les tensioactifs convenant à la mise en oeuvre de la présente invention sont notamment les suivants :

### (i) Tensioactif(s) anionique(s) :

Leur nature ne revêt pas, dans le cadre de la présente invention, de caractère véritablement critique.

Ainsi, à titre d'exemple de tensio-actifs anioniques utilisables, seuls ou mélanges, dans le cadre de la présente invention, on peut citer notamment (liste non limitative) les sels (en particulier sels alcalins, notamment de sodium, sels d'ammonium, sels d'amines, sels d'aminoalcools ou sels de magnésium) des composés suivants : les alkylsulfates, les alkyléthersulfates, alkylamidoéthersulfates, alkylarylpolyéthersulfates, monoglycérides sulfates ; les alkylsulfonates, alkylphosphates, alkylamidesulfonates, alkylarylsulfonates, α-oléfine-sulfonates, paraffine-sulfonates ; les alkylsulfosuccinates, les alkyléthersulfosuccinates, les alkylamidesulfosuccinates; les alkylsulfosuccinamates ; les alkylsulfoacétates ; les alkylétherphosphates; les acylsarcosinates ; les acyliséthionates et les N-acyltaurates, le radical alkyle ou acyle de tous ces différents composés comportant de préférence de 12 à 20 atomes de carbone, et le radical aryl désignant de préférence un groupement phényle ou benzyle. Parmi les tensioactifs anioniques encore utilisables, on peut également citer les sels d'acides gras tels que les sels des acides oléique, ricinoléique, palmitique, stéarique, les acides d'huile de coprah ou d'huile de coprah hydrogénée ; les acyl-lactylates dont le radical acyle comporte 8 à 20 atomes de carbone. On peut également utiliser des tensioactifs faiblement anioniques, comme les acides d'alkyl D galactoside uroniques et leurs sels ainsi que les acides éthers carboxyliques polyoxyalkylénés et leurs sels, en particulier ceux comportant de 2 à 50 groupements oxyde d'éthylène, et leurs mélanges. Les tensioactifs anioniques du type acides ou sels d'éthers carboxyliques polyoxyalkylénés sont en particulier ceux qui répondent à la formule (1) suivante : 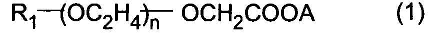 dans laquelle :
R₁ désigne un groupement alkyle alkylamido ou alkaryle, et n est un nombre entier ou décimal (valeur moyenne) pouvant varier de 2 à 24 et de préférence de 3 à 10, le radical alkyle ayant entre 6 et 20 atomes de carbone environ, et aryle désignant de préférence phényle,

A désigne H, ammonium, Na, K, Li, Mg ou un reste monoéthanolamine ou triéthanolamine. On peut également utiliser des mélanges de composés de formule (1) en particulier des mélanges dans lesquels les groupements R₁ sont différents.

Des composés de formule (1) sont vendus par exemple par la Société CHEM Y sous les dénominations AKYPO (NP40, NP70, OP40, OP80, RLM25, RLM38, RLMQ 38 NV, RLM 45, RLM 45 NV, RLM 100, RLM 100 NV, RO 20, RO 90, RCS 60, RS 60, RS 100, RO 50) ou par la Société SANDOZ sous les dénominations SANDOPAN (DTC Acid, DTC).

### (ii) Tensioactif(s) non ionique(s) :

Les agents tensioactifs non-ioniques sont, eux aussi, des composés bien connus en soi (voir notamment à cet égard "Handbook of Surfactants" par M.R. PORTER, éditions Blackie & Son (Glasgow and London), 1991, pp 116-178) et leur nature ne revet pas, dans le cadre de la présente invention, de caractère critique. Ainsi, ils peuvent être notamment choisis parmi (liste non limitative) les alcools gras polyéthoxylés, polypropoxylés ou polyglycérolés, les alpha-diols gras polyéthoxylés, polypropoxylés ou polyglycérolés, les alkylphénols gras polyéthoxylés, polypropoxylés ou polyglycérolés et les acides gras polyéthoxylés, polypropoxylés ou polyglycérolés, tous ayant une chaîne grasse comportant par exemple 8 à 18 atomes de carbone, le nombre de groupements oxyde d'éthylène ou oxyde de propylène pouvant aller notamment de 2 à 50 et le nombre de groupements glycérol pouvant aller notamment de 2 à 30. On peut également citer les copolymères d'oxyde d'éthylène et de propylène, les condensats d'oxyde d'éthylène et de propylène sur des alcools gras ; les amides gras polyéthoxylés ayant de préférence de 2 à 30 moles d'oxyde d'éthylène, les amides gras polyglycérolés comportant en moyenne 1 à 5 groupements glycérol et en particulier 1,5 à 4 ; les esters d'acides gras du sorbitan oxyéthylénés ayant de 2 à 30 moles d'oxyde d'éthylène ; les esters d'acides gras du sucrose, les esters d'acides gras du polyéthylèneglycol, les alkylpolyglycosides, les dérivés de N-alkyl glucamine, les oxydes d'amines tels que les oxydes d'alkyl (C₁₀ - C₁₄) amines ou les oxydes de N-acylaminopropylmorpholine. On notera que les alkylpolyglycosides constituent des tensio-actifs non-ioniques rentrant particulièrement bien dans le cadre de la présente invention.

### (iii) Tensioactif(s) amphotère(s) ou zwittérionique(s) :

Les agents tensioactifs amphotères ou zwitterioniques, dont la nature ne revêt pas dans le cadre de la présente invention de caractère critique, peuvent être notamment (liste non limitative) des dérivés d'amines secondaires ou tertiaires aliphatiques, dans lesquels le radical aliphatique est une chaîne linéaire ou ramifiée comportant 8 à 18 atomes de carbone et contenant au moins un groupe anionique hydrosolubilisant (par exemple carboxylate, sulfonate, sulfate, phosphate ou phosphonate) ; on peut citer encore les alkyl (C₈-C₂₀) bétaïnes, les sulfobétaïnes, les alkyl (C₈-C₂₀) amidoalkyl (C₁-C₆) betaïnes ou les alkyl (C₈-C₂₀) amidoalkyl (C₁-C₆) sulfobétaïnes.

Parmi les dérivés d'amines, on peut citer les produits vendus sous la dénomination MIRANOL, tels que décrits dans les brevets US-2 528 378 et US-2 781 354 et classés dans le dictionnaire CTFA, 3ème édition, 1982, sous les dénominations Amphocarboxyglycinates et Amphocarboxypropionates de structures respectives :

R₂ -CONHCH₂CH₂ -N⁺(R₃)(R₄)(CH₂COO⁻) (2)

dans laquelle : R₂ désigne un radical alkyle d'un acide R₂-COOH présent dans l'huile de coprah hydrolysée, un radical heptyle, nonyle ou undécyle, R₃ désigne un groupement bêta-hydroxyéthyle et R₄ un groupement carboxyméthyle ;
et

R₂'-CONHCH₂CH₂-N(B)(C) (3)

dans laquelle :
B représente -CH₂CH₂OX', C représente -(CH₂)_{z} -Y', avec z = 1 ou 2,
X' désigne le groupement -CH₂CH₂-COOH ou un atome d'hydrogène
Y' désigne -COOH ou le radical -CH₂ - CHOH - SO₃H
R₂' désigne un radical alkyle d'un acide R₉ -COOH présent dans l'huile de coprah ou dans l'huile de lin hydrolysée, un radical alkyle, notamment en C₇, C₉, C₁₁ ou C₁₃, un radical alkyle en C₁₇ et sa forme iso, un radical C₁₇ insaturé.

A titre d'exemple on peut citer le cocoamphocarboxyglycinate vendu sous la dénomination commerciale MIRANOL C2M concentré par la Société MIRANOL.

Dans les compositions conformes à l'invention, on prérère utiliser au moins un tensioactif anioniques.

Dans les compositions conformes à l'invention, on peut utiliser des mélanges d'agents tensioactifs et en particulier des mélanges d'agents tensioactifs anioniques, des mélanges d'agents tensioactifs anioniques et d'agents tensioactifs amphotères et/ou non ioniques. Un mélange particulièrement préféré est un mélange comprenant au moins un agent tensioactif anionique et au moins un agent tensioactif amphotère.

La quantité d'agents tensioactifs anioniques va de préférence de 3 % à 40% en poids, rapportée au poids total de la composition cosmétique. Elle va de préférence de 5 % à 25 % en poids et, mieux encore, de 8 % à 20 % en poids.

La quantité d'agents tensioactifs amphotères et/ou non ioniques, lorsqu'ils sont présents, va de préférence de 0,5 à 20 %, et en particulier de 1 à 15 % rapportée au poids total de la composition.

Le milieu cosmétiquement acceptable est un milieu comprenant de l'eau ou de l'eau et au moins un solvant organique cosmétiquement acceptable.
Par solvant organique, on entend au sens de la présente invention un composé organique liquide à la température de 25°C et à la pression atmosphérique. De préférence, le composé organique est polaire.

De préférence, le solvant est un alcool. Celui-ci est notamment choisi parmi les alcools inférieurs en C₁-C₄ comme l'éthanol, l'isopropanol, le tertio-butanol, le n-butanol ; les polyols comme le propylèneglycol, les éthers de polyols et leurs mélanges, l'alcool particulièrement préféré étant l'éthanol.

Dans le milieu cosmétiquement acceptable, la proportion en eau peut être comprise entre 50 et 95% en poids par rapport au poids total de la composition et plus particulièrement de 75 à 95.
Les solvants peuvent être présent dans des concentrations allant généralement de 1 à 45% en poids par rapport au poids total de la composition.

La composition cosmétique détergente selon l'invention peut contenir en outre au moins un additif choisi parmi les silicones sous forme soluble, dispersée, micro-dispersée, les agents tensio-actifs cationiques, les céramides et pseudo-céramides, les agents apaisants, les agents anti-pelliculaires tels que le Climbazole, le Piroctone Olamine, la Pyridinethione de Zinc, le sulfure de sélénium, l'acide undécylénique et ses sels, les polymères non-ioniques, cationiques, amphotères, anioniques et leurs sels, de type statistique branché ou non, les agents structurants des cheveux, les agents chélateurs de minéraux, les réducteurs, les oxydants, les vitamines et pro-vitamines dont le panthénol, les huiles végétales, animales, minérales et synthétiques, les cires autres que les céramides et pseudo-céramides, les filtres solaires hydrosolubles et liposolubles, siliconés ou non siliconés, le glycérol, les pigments minéraux et organiques, colorés ou non colorés, les colorants permanents ou temporaires, les agents nacrants et opacifiants, les agents séquestrants, les agents plastifiants, les agents solubilisants, les agents acidifiants, des agents alcalinisants, les agents anti-oxydants, les hydroxyacides, les agents de pénétration, les parfums, les agents solubilisants du parfum (peptisant), les agents conservateurs, les agents anti-corrosion, les agents rubéfiants et les actifs traitants tels que les agents anti-chute des cheveux dont l'Aminexil, et les mélanges de ces composés.

L'homme de métier veillera à choisir les éventuels additifs et leur quantité de manière à ce qu'ils ne nuisent pas aux propriétés des compositions de la présente invention.
Ces additifs peuvent être présents dans la composition cosmétique selon l'invention en une quantité allant de 0,000001 à 20 % en poids par rapport au poids total de la composition cosmétique.

La composition peut contenir en outre un additif choisi parmi les conservateurs, les agents de régulation de pH, les parfums.

Le pH des compositions utilisées selon l'invention est généralement compris entre 2 et 12, de préférence de 3 à 8 et plus particulèrement de 5 à 7.

La composition est dispensée sur les cheveux et/ou le cuir chevelu de préférence sous forme de gouttelettes. Les gouttelettes ont de préférence un volume moyen allant de 10 à 500 microlitre et plus particulièrement de 50 à 200 microlitre.

L'application de la composition peut se faire avec les mains, avec un embout applicateur, avec un peigne dispensateur.

L'application peut également se faire à l'aide d'un dispositif permettant de vaporiser (ou pulvériser) la composition sous forme de goutelettes. Il peut s'agir par exemple d'un flacon-pompe ou d'un dispositif aérosol.

L'invention a également pour objet un flacon-pompe comprenant une composition cosmétique liquide comprenant au moins 5% d'au moins un tensioactif détergent.

L'invention a également pour objet un dispositif aérosol comprenant au moins un propulseur et une composition cosmétique liquide comprenant au moins 5% d'au moins un tensioactif détergent.

L'invention vise également un procédé pour le lavage des cheveux frisés ou crépus dans lequel ces dispositifs sont mis en oeuvre.

Dans les exemples qui suivent, donnés à titre illustratif et non limitatif, on donne des compositions concrètes conformes à l'invention.

### EXEMPLE 1 :

Une composition A selon l'invention contenant 13 % de Polyglycéryl-3-Hydroxylaurylether, de viscosité 9 Centipoises (mesurée à 25°C et à un taux de cisaillement de 1s⁻¹, a été comparée à une composition B du commerce épaisse, de viscosité 9500 Centipoises (Shampooing neutralisant pour cheveux sensibilisés de GOLDYS).
Le temps nécessaire au démarrage de la mousse et au rinçage de la composition ont été mesurés sur un panel de modèles à l'aide d'un chronomètre.

| | A | B |
|---|---|---|
| Durée du démarrage | 2 à 10 secondes | 15 à 30 secondes |
| Durée du rinçage | Environ 1 minute | 2 à 3 minutes |

### Etude du cuir chevelu lavé :

Deux petites zones de 1 cm² du cuir chevelu sont dénudées par demi-tête, en coupant les cheveux à ras du cuir chevelu afin d'avoir accès à cette dite surface. Sur l'une des 2 zones de chaque demi-tête, on utilisera un appareil de détection et de quantification des lipides sébacés tel que le Sébumètre de la Société Courage et Khazaka . Sur l'autre zone de chaque demi-tête, on appliquera une bande adhésive telle que le D'Squame, vendue par la Société Cutec, afin de prélever puis révéler et de quantifier la présence et la densité des levures Malassezia encore présentes, selon la technique publiée par G E Piérard and coll dans l'article « Correlation between Malassezia Ovalis load and Dandruff severity. *J Mycol Med.* 8, 83-86, 1998 ».
La réalisation couplée de ces 2 déterminations montre clairement une quasi absence de sébum résiduel dans la zone lavée par la composition A selon l'invention alors que le sébum est présent à des taux élevés dans celle lavée avec la composition B de forte viscosité. Il en est de même pour la colonisation des Malassezia qui chute nettement dans le cas de la composition selon l'invention de basse viscosité.

Après séchage, la chevelure lavée avec la composition selon l'invention est plus nette, plus aérée que celle traitée avec la composition B. Le toucher est également plus agréable avec la composition selon l'invention.

## Revendications

1. Procédé de nettoyage des cheveux bouclés, frisés ou crépus et du cuir chevelu correspondant, ledit procédé étant **caractérisé par le fait qu'**il comprend les étapes suivantes :
(i) on applique sur les cheveux secs ou humides une composition liquide comprenant dans un milieu cosmétiquement acceptable au moins 5% d'au moins un tensioactif détergent,
(ii) on masse les cheveux et le cuir chevelu éventuellement à l'aide des doigts ,
(iii) on rince les cheveux avec de l'eau,
la viscosité de la composition allant de 1 à 100 mPa/s.

2. Procédé selon la revendication 1, **caractérisée en ce que** la viscosité de la composition se situe de 2 à 20 mPa/s.

3. Procédé selon l'une des revendications 1 ou 2, **caractérisée en ce que** le tensioactif détergent est choisi parmi les tensioactifs anioniques, amphotères, non ioniques et zwittérioniques et leurs mélanges.

4. Procédé selon l'une quelconque des revendications 1 à 3, **caractérisée en ce que** les tensioactifs détergents sont choisis parmi les tensioactifs anioniques.

5. Procédé selon l'une quelconque des revendications 1 à 4, **caractérisée en ce que** les tensioactifs détergents sont choisis parmi les mélanges de tensioactifs anioniques et de tensioactifs non ioniques et/ou amphotères.

6. Procédé selon l'une quelconque des revendications 1 à 5, **caractérisée en ce que** les tensioactifs détergents représentent de 5 % à 50 % en poids, de préférence de 6 % à 30 % en poids, et encore plus préférentiellement de 8 % à 25 % en poids, du poids total de la composition finale.

7. Procédé selon l'une quelconque des revendications 1 à 6, **caractérisée en ce que** les tensioactifs anioniques sont présents de 3 % à 40% en poids, par rapport au poids total de la composition, de préférence de 5 % à 25 % en poids et, mieux encore, de 8 % à 20 % en poids.

8. Procédé selon l'une quelconque des revendications 1 à 7, **caractérisée en ce que** la quantité d'agents tensioactifs amphotères et/ou non ioniques, lorsqu'ils sont présents, va de 0,5 à 20 %, et en particulier de 1 à 15 % par rapport au poids total de la composition.

9. Procédé selon l'une quelconque des revendications 1 à 8, **caractérisée en ce que** la composition comprend en outre au moins un adjuvant choisi parmi les silicones sous forme soluble, dispersée, micro-dispersée, les agents tensio-actifs cationiques, les céramides et pseudo-céramides, les agents apaisants, les agents anti-pelliculaires tels que le Climbazole, le Piroctone Olamine, la Pyridinethione de Zinc, le sulfure de sélénium, l'acide undécylénique et ses sels, les polymères non-ioniques, cationiques, amphotères, anioniques et leurs sels, de type statistique branché ou non, les agents structurants des cheveux, les agents chélateurs de minéraux, les réducteurs, les oxydants, les vitamines et pro-vitamines dont le panthénol, les huiles végétales, animales, minérales et synthétiques, les cires autres que les céramides et pseudo-céramides, les filtres solaires hydrosolubles et liposolubles, siliconés ou non siliconés, le glycérol, les pigments minéraux et organiques, colorés ou non colorés, les colorants permanents ou temporaires, les agents nacrants et opacifiants, les agents séquestrants, les agents plastifiants, les agents solubilisants, les agents acidifiants, des agents alcalinisants, les agents anti-oxydants, les hydroxyacides, les agents de pénétration, les parfums, les agents solubilisants du parfum (peptisant), les agents conservateurs, les agents anti-corrosion, les agents rubéfiants et les actifs traitants tels que les agents anti-chute des cheveux dont l'Aminexil, et les mélanges de ces composés.

10. Procédé selon l'une quelconque des revendications 1 à 9, **caractérisée en ce que** le milieu cosmétiquement acceptable est un milieu comprenant de l'eau ou de l'eau et au moins un solvant organique cosmétiquement acceptable.

11. Procédé selon l'une quelconque des revendications 1 à 10, **caractérisée en que** ladite composition est dispensée sous forme de gouttelettes.

12. Utilisation d'une composition détergente liquide telle que définie à l'une des revendications 1 à 11 pour nettoyer les cheveux bouclés, frisés ou crépus et le cuir chevelu correspondant.

13. Utilisation d'une composition liquide ayant une viscosité allant de 1 à 100 mPa/s comprenant dans un milieu cosmétiquement acceptable au moins 5% d'au moins un tensioactif détergent,selon le procédé de la revendication 1 sur des cheveux bouclés, frisés ou crépus pour obtenir un bon démarrage de mousse et/ou une meilleure réduction des quantités résiduelles de sébum et/ou une meilleure réduction de la flore résidente.

14. Dispositif permettant de vaporiser (ou pulvériser) une composition sous forme de goutelettes comprenant une composition liquide ayant une viscosité allant de 1 à 100 mPa/s comprenant dans un milieu cosmétiquement acceptable au moins 5% d'au moins un tensioactif détergent.

15. Dispositif selon la revendication 14, **caractérisée en ce que** le dispositif est un flacon-pompe.

16. Dispositif selon la revendication 14, **caractérisée en ce que** le dispositif est un dispositif aérosol.
